# EUROPÄISCHE PATENTSCHRIFT

(11) **EP 3 569 186 B1**
(45) Veröffentlichungstag und Bekanntmachung des Hinweises auf die Patenterteilung: **12.05.2021**
(21) Anmeldenummer: 19182870.6
(22) Anmeldetag: 08.07.2016
(51) Int. Cl.: A61C 9/00, A61C 13/00

(54) **VERFAHREN ZUR HERSTELLUNG EINER PROTHESE**
METHOD FOR PRODUCING A PROSTHESIS
PROCÉDÉ DE FABRICATION D'UNE PROTHÈSE

(43) Veröffentlichungstag der Anmeldung: 20.11.2019
(62) Teilanmeldung aus: 16178660.3
(73) Patentinhaber: Ivoclar Vivadent AG, 9494 Schaan (LI)
(72) Erfinder: Frei, Christian, 39025 Naturns (IT); Watzke, Ronny, 6800 Feldkirch (AT)
(74) Vertreter: Baronetzky, Klaus

(56) Entgegenhaltungen:
- WO-A1-2015/089676
- DE-A1-102012 111 020
- DE-A1-102013 102 421
- DE-T2- 69 935 969
- US-A- 5 961 325
- US-A1- 2009 117 514
- US-A1- 2016 008 106

## Beschreibung

Die Erfindung betrifft ein Verfahren zur Herstellung einer Prothese, gemäß dem Anspruch 1.

Es ist seit langem bekanntgeworden, für die Erfassung der Mundsituation eines Patienten sogenannte Abformlöffel zu verwenden. Hier haben sich verschiedene Systeme etabliert.

Abgesehen von der einfachen und statischen Abformung beispielsweise eines Kieferkammes ist in vielen Fällen auch der Bezug zum gegenüberliegenden Kiefer relevant. Hierzu ist es bekanntgeworden, entsprechend geeignete Abformlöffel für Oberkiefer und Unterkiefer je getrennt vorzunehmen und die Relativposition über Registrierstifte, über die sogenannte Bissregistrierung, vorzunehmen.

Ferner ist es bekannt, an die Kieferkammbögen jeweils Wachswälle anzuschließen, um so die Relativposition von Oberkiefer und Unterkiefer zu erfassen, beziehungsweise festzulegen.

Ein Beispiel für eine derartige Lösung ist aus der DE 20 218 063 U1 ersichtlich

Dokument WO 2015/089676 A1 offenbart ein Verfahren zur Erfassung erster Daten eines individuellen Ober- und Unterkieferbogens zur Modellierung des Ober- und Unterkieferbogens. Es wird ein Paar von Komponenten bereitgestellt, um den Ober- und Unterkieferbogen in einer maxillomandibularen Beziehung zu erhalten, die die Bissposition definiert. Das Komponentenpaar ist mit Öffnungen zur Freilegung des durchgehenden Wegs zwischen Ober- und Unterkieferbogen definiert. Zweite Daten werden entlang des kontinuierlichen Wegs von Ober- und Unterkieferbogen in Bissposition erfasst, um die Position von Ober- und Unterkieferbogen in Bissposition zu modellieren.

Herr Josephus Schreinemaker brachte vor längerer Zeit Abformlöffel auf den Markt, deren Besonderheit in der Autoklavierbarkeit liegt. So sind Abformlöffel bekanntgeworden, die aus einer mit Durchbrechungen versehenen Metallbasis bestehen, auf welcher Metallbasis die Abformmasse aufgebracht ist. Hierzu ist eine Trägerplatte des Abformlöffels metallisch und daher temperaturbeständig beispielsweise bei 120 oder 140 Grad Celsius. Über Durchbrechungen lässt sich das eingebrachte Abformmaterial, dass beispielsweise wachsartig sein kann, in dem Abformlöffel verankern.

Wenn eine Funktionsabformung erwünscht ist, muss der Abformlöffel geführt werden, so dass ein Löffelgriff vorgesehen ist, der durch den Mund hindurch verläuft. Dies wird vom Patienten teilweise als unangenehm empfunden; zudem hängt die Qualität der Abformung stark von der Person ab, die die Abformung vornimmt. Bei einem Löffelgriff wird die Funktionsabformung aufgrund des mundoffenen Verfahrens und durch die Finger des Behandlers mitunter negativ beeinflusst.

Demgegenüber liegt der Erfindung die Aufgabe zugrunde, ein Verfahren zur Herstellung einer Prothese gemäß dem Anspruch 1 zu schaffen, die für die Bereitstellung einer besser angepassten Prothese geeignet sind, und zugleich eine bessere Akzeptanz beim Patienten ermöglicht.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, dass die Abformlöffel aus den zugehörigen Kieferkammbogen und Wachswällen bestehen, die zueinander einstückig ausgebildet sind.

Bei Bedarf lässt sich der so realisierte Abformlöffel dann in seiner Form anpassen. Der Abformlöffel ist bei einer Temperatur von deutlich mehr als der Mundtemperatur verformbar, beispielsweise 60,80 oder unter 100 Grad, und zwar mit einer Norm-Handkraft, die weniger als 100 N oder weniger als 50 N beträgt.

Ein besonderer Vorteil des Kombination des Abformlöffels mit dem Referenzmuster, das im Frontbereich auf diesem aufgebracht ist, ist, dass die Behandlung des Patienten auf 2 Termine reduzierbar ist: Zum einen die Abformung und Datenerfassung, zum anderen das Einsetzen der neuen Prothesen. Aufgrund der besseren Anpassbarkeit müssen auch für unterschiedliche Größen weniger verschiedene Abformlöffel bereitgehalten werden, beispielsweise lediglich eine kleine, eine mittlere und eine große Größe, so dass die Erstauswahl des betreffenden Abformlöffels oder Wachslöffels auch ohne Vermessung auf Sicht erfolgen kann.

Die Verformung kann beispielsweise so erfolgen, dass der Abformlöffel in ein warmes Wasserbad gelegt wird, das eine Erwärmung auf beispielsweise 50 oder 60 Grad und ein gegebenenfalls erforderlichen Verformen extraoral ermöglicht.

Die Verformung erfolgt bevorzugt in bukkal-oraler Richtung, wobei es sich versteht, dass auch in Anpassung an die Patientenanatomie eine partielle Vertikalverformung, also in okklusal-gingivaler Richtung, für jede Seite des Kieferkammbogens unabhängig von der anderen möglich ist.

Die insofern vorbereiteten Kieferkammbögen mit integrierten Wachswällen werden nun intraoral überprüft, und es wird an den Okklusionsseiten der Bisswälle festgestellt, ob das Aufeinanderliegen dieser spaltfrei möglich ist. Wenn ein Spalt sichtbar ist, werden überschüssige Bereiche mit einem hierzu geeigneten Werkzeug, beispielsweise einem sogenannten Rimformer, abgetragen, bis beide Okklusionsseiten der Wachswälle plan aufeinander aufliegen.

Die Verformung, wie sie hier erläutert wird, bezieht sich auf die plastische Verformbarkeit. Es versteht sich, dass je nach Materialwahl zusätzlich zur plastischen Verformbarkeit auch eine elastische Mikroverformbarkeit vorliegt. Der erfindungsgemäße Abformlöffel federt in diesem Fall beim Verformen ganz leicht zurück, wobei es bevorzugt ist, die elastische Verformbarkeit sehr gering zu halten, zumindest im Vergleich zur tatsächlich erfolgten plastischen Verformung.

Das genannte Verhältnis wird wesentlich von der Materialwahl, aber auch der Verformungstemperatur bestimmt. Wenn beispielsweise eine typisches Dentalwachs auf hohe Temperaturen wie 80 oder 85 Grad erwärmt wird, ist die elastische Verformbarkeit nahezu entfallen. Bei diesen Temperaturen ist allerdings die Formtreue nicht in allen Umständen gewährleistet, so dass es bevorzugt ist, die Verformungstemperatur so zu wählen, dass die elastische Verformbarkeit nicht mehr merkbar besteht, jedoch die Formtreue erhalten bleibt.

Nach der planen Ausrichtung der Wachswälle erfolgt die Funktionsabformung bei geschlossenem Mund. Die beiden Abformlöffel oder Kieferkammbögen mit integrierten Wachswällen werden bei geschlossenem Mund durch einen entsprechenden durch den Patienten auszuübenden Druck aufeinander gedrückt, nachdem sie mit einem geeigneten Abformmaterial gefüllt worden sind.

Das Abformmaterial wird hierdurch verformt.

Hieran anschließend wird bevorzugt ein Okklusionom, also ein Gesichtsbogen mit angeformter Bissgabel, so positioniert, dass die Bissgabel zwischen beiden Wachswällen zu liegen kommt. Die Okklusionsebene ist so definiert, und die so erfassten Werte werden notiert.

Besonders bevorzugt ist es, wenn der Oberkiefer-Wachswall an seinem distalen Ende eine Abschrägung aufweist.

Hieran anschließend können bei geöffneten Mund des Patienten bei Bedarf Ästhetiklinien, also mindestens Lippenschlusslinien und Mittellinien, ggf. auch Lachlinien, im labialen Bereich eines Wachswalls, insbesondere des Oberkiefers-Wachswalls angezeichnet werden, und es werden dort Referenzmuster aufgebracht, die bei geöffneten Lippen, also beim Lächeln des Patienten, vollständig sichtbar sind.

Die Referenzmuster können in beliebiger geeigneter Weise ausgestaltet sein:
Es ist beispielsweise möglich, einen entsprechenden Aufkleber anzubringen; es kann eine entsprechende Wachsform aufgebracht werden, oder es kann über einen Stempel ein entsprechendes Wachsmuster in den Wachswall kalt oder heiß eingebracht werden.

Es ist auch möglich, dass das Referenzmuster bereits auf dem Kieferkammbogen mit integriertem Wachswall vorhanden ist.

Ferner besteht auch die Möglichkeit, dass das Referenzmuster auf einem aus Kunststoff gefrästem Abformlöffel bereits vorhanden ist.

Es wird nun ein 2-dimensionales Bild des Mundes des Patienten mit gespreizten Lippen erstellt, wobei die Referenzkörper des Referenzmusters sämtlich sichtbar sein sollen.

Für die hieran anschließende Bissregistrierung kann beispielsweise ein Verschlüsselungsmaterial zwischen die Okklusionsebenen der Wachswälle gebracht werden. Alternativ kann ein Stützstiftregistrat, beispielsweise das Gnathometer CAD der Fa. Wieland Dental, Pforzheim, in mindestens einen Wachswall heiß eingedrückt werden und so die Kieferkammbögen gegeneinander verschlüsseln.

Die verschlüsselten Kieferkammbögen mit integrierten Wachswällen werden nun gemeinsam, also in verschlüsselter Form, von einer Scannvorrichtung 3-dimensional gescannt, und die Okklusionsebene, die auch basierend auf den Werten des Okklusionoms erfasst wurde, wird von einer Verarbeitungsvorrichtung in die Scandaten eingebracht.

Anschließend heran erfolgt durch die Verarbeitungsvorrichtung die virtuelle Zahnaufstellung, wobei die Zähne in an sich bekannter Weise nach zahntechnischen Regeln, gegebenenfalls unter Beachtung der aufgebrachten Ästhetiklinien, angeordnet werden.

Anschließend hieran wird das zuvor aufgenommene Bild des Referenzmusters mit dem um dieses herum sichtbaren Lippen des Patienten, das auch als Lächelbild bezeichnet wird, mit der virtuellen Zahnaufstellung zusammengeführt und in Übereinstimmung gebracht. Die Zusammenführung erfolgt zwischen dem 2D-Lächelbild und den 3D-Scandaten der Abformung einschließlich des gescannten Referenzmusters.Die Ausrichtung und die Höhenlage der Oberkiefer-Schneidezähne - und damit indirekt sämtlicher Zähe 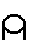 wird so perfekt entsprechend dem Lächelbild realisiert, was erhebliche ästhetische Vorteile gegenüber einer rein funktionsbezogenen Aufstellung bietet.

Die CAD-Daten werden basierend hierauf, also nach Anpassung der Lage der Zähne an das "Lächelbild", bereitgestellt, und die Zähne werden nun in geeigneter Weise gefertigt, beispielsweise durch auftragende oder abtragende Verfahren.hergestellt. Eine Anpassung erfolgt nun ggf., grundsätzlich werden die Zähne basierend auf den Ästhetiklinien aufgestellt.

Weitere Einzelheiten, Vorteile und Merkmale ergeben sich aus der Beschreibung der nachstehenden Ausführungsbeispiele der Erfindung anhand der Zeichnung.

### Es zeigen:

- Fig. 1: eine Abbildung mehrerer Abformlöffel in erfindungsgemäßer Ausgestaltung, je bestehend aus einem Unterkiefer-Abformlöffel und einem Oberkiefer-Abformlöffel, und je in unterschiedlichen Größen;
- Fig. 2: eine perspektivische Darstellung einer vollständigen Anordnung eines Abformlöffels, der für die Aufnahme von Abdruckmasse bereit ist;
- Fig. 3: eine Darstellung eines Oberkiefer- und eines Unterkiefer-Abformlöffels, wobei unter Einsatz eines Rimformers ein klaffender Spalt vermieden wird;
- Fig. 4: eine Ausführungsform eines Oberkiefer-Abformlöffels mit aufgebrachtemAbformmaterial;
- Fig. 5: eine distale Ansicht einer verschlüsselten Abformlöffel-Anordnung mit aufgebrachter Abdruckmasse und genommenen Abdruck;
- Fig. 6: ein 2-dimensionales Bild eines geöffneten Mundes mit eingeblendetem Oberkiefer-Abformlöffel, soweit sichtbar, unter Darstellung eines Referenzmusters;
- Fig. 7: eine Frontansicht einer virtuellen Darstellung des Oberkiefer- und des Unterkiefer-Abformlöffels mit aufgebrachten Referenzmuster;
- Fig. 8: eine perspektivische Darstellung eines Oberkiefer-Abformlöffels in einer weiteren Ausführungsform;
- Fig. 9: eine perspektivische Darstellung eines Unterkiefer-Abformlöffels in einer weiteren Ausfürungsform; und
- Fig. 10: eine perspektivische Darstellung eines Oberkiefer- und eines Unterkiefer-Abformlöffels in einer weiteren Ausfürungsform.

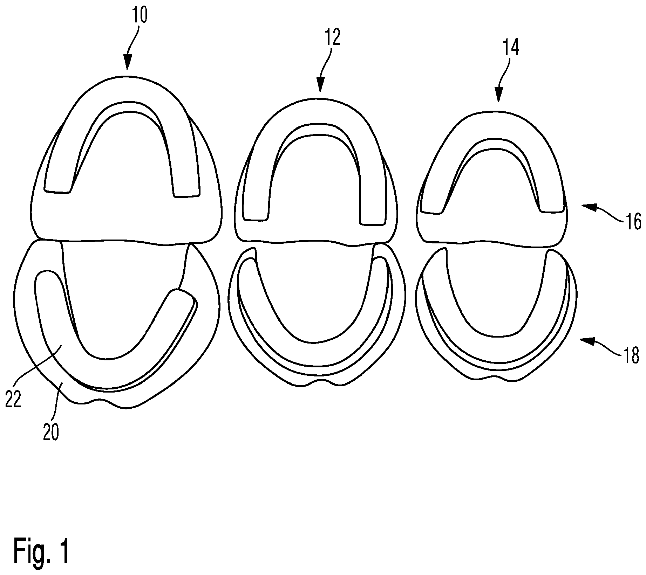

Aus Fig. 1 ist eine mögliche Ausgestaltung von Abformlöffeln ersichtlich. Es handelt sich hier um Anordnungen mit einem großen Abformlöffel 10, einem mittleren 12 und einem kleinen 14. Diese bestehen je aus einem Oberkiefer-Abformlöffel 16 und einem Unterkiefer-Abformlöffel 18. Jeder Abformlöffel besteht aus einem Kieferkammbogen, der für die Aufnahme von Abformmasse für die Abdrucknahme bestimmt ist, und je einem Wachswall 22, wobei Kieferkammbogen 20 und Wachswall 22 einstückig aneinander angeformt sind.

Für die Unterkiefer-Abformbögen verlaufen die Wachswälle und Kieferkammbögen 22 und 20 je entsprechend dem Kieferkamm, also im wesentlichen U-förmig.

Für die Oberkiefer-Abformlöffel 16 ist zusätzlich ein Gaumenschild 24 vorgesehen, der sich zwischen den beiden Enden des betreffenden Oberkiefer-Kammbogens erstreckt, wie es an sich bekannt ist.

Die Abformlöffel bestehen aus einem Material mit einem Erweichungspunkt zwischen 40 und etwa 100 Grad Celsius. Für die Anpassung an den betreffenden Kieferkamm des Patienten lassen sie sich dementsprechend erhöhter Temperatur - beispielsweise nach Entnahme aus einem Warmwasserbad - in geeigneter Weise verformen.

Die Bereitstellung von drei Größen von Abformlöffeln ermöglicht es gepaart mit dieser Maßnahme, sämtliche in der Praxis vorkommenden Kieferformen von Patienten abzudecken.

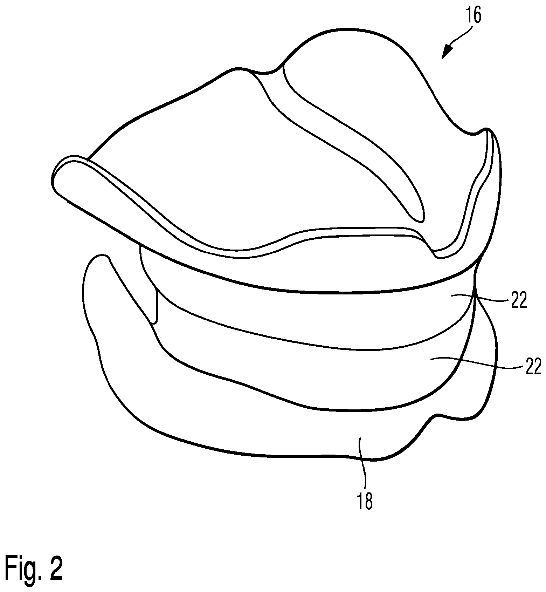

Aus Fig. 2 ist eine Anordnung aus einem Oberkiefer-Abformlöffel 16 und einem Unterkiefer-Abformlöffel 18 ersichtlich. In der dargestellten Ausgestaltung fluchten die Wachswälle miteinander und liegen ohne Spalt mit Ihren Okklusionsflächen aneinander an.

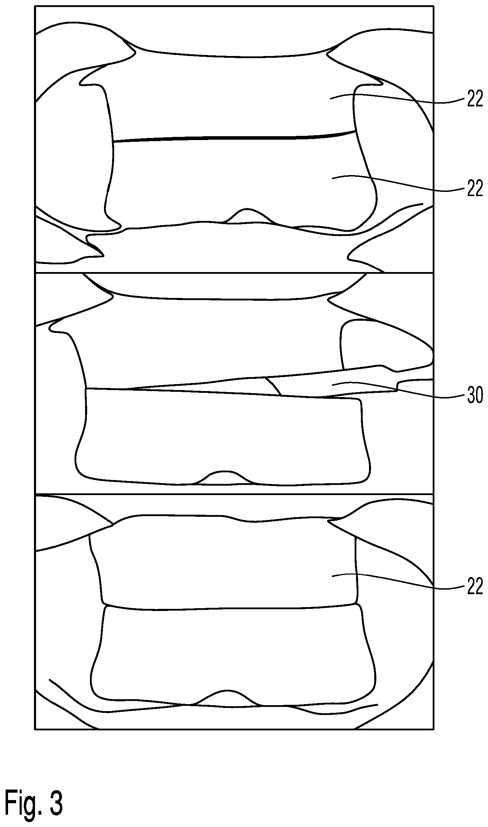

Fig. 3 zeigt Maßnahmen, die eine spaltfreie Okklusion der Wachswälle 22 miteinander ermöglichen. Wenn ein einseitiger Spalt 30 entsteht, wie es aus dem mittleren Bild aus Fig. 3 ersichtlich ist, lässt sich dieser über ein entsprechendes Werkzeug füllen, so dass wiederum eine im wesentlichen spaltfreier Anlage entsteht, wie es aus dem unteren Bild von Fig. 3 ersichtlich ist.

Alternativ lässt sich auch an der in der Darstellung linken Seite der Wachswälle Material abtragen.

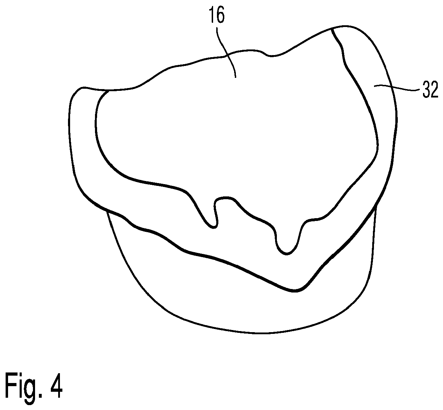

Aus Fig. 4 ist ein Abformlöffel 16 ersichtlich, dessen Kieferkamm-Nuten bereits mit Abformmaterial 32 gefüllt sind. Das Füllen kann in jeglicher geeigneter Weise erfolgen, wobei die Bereitstellung eines entsprechenden Abformmaterial-Überschuss es ermöglicht, dass sämtliche Einzelheiten der Kieferkämme abgeformt werden können.

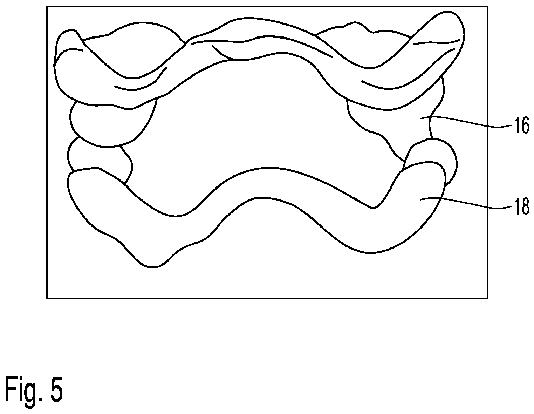

Eine distale Ansicht von Abformlöffeln 16 und 18 ist aus Fig. 5 zu entnehmen. Dort ist die Abformung bereits erfolgt und die verschlüsselten Abformlöffel sind aus dem Mund des Patienten entnommen, so dass sie gescannt werden können.

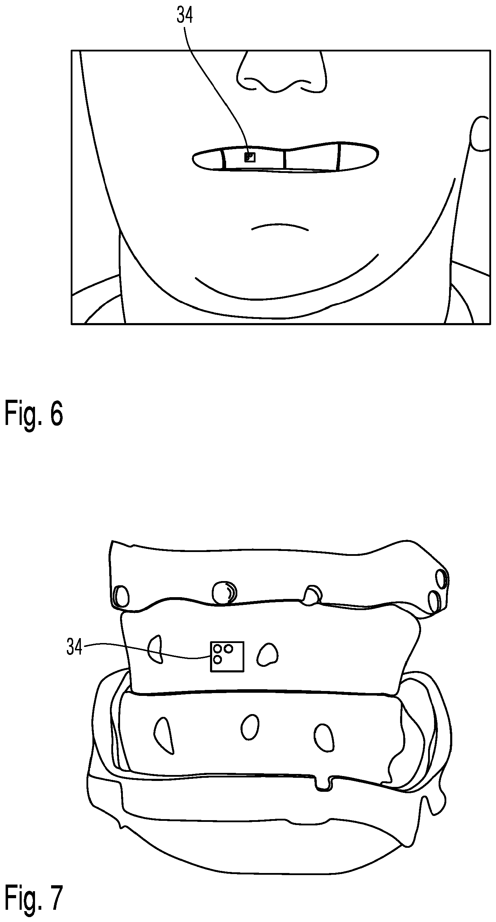

Aus Fig. 6 ist ein so genanntes Lächelbild einer Patientin bzw. eines Patienten ersichtlich. Der Patient öffnet die Lippen, so dass die Oberkieferzähne - oder bei einem Zahnlosen Patienten die Oberkiefer - sichtbar sind.

Der Zahnarzt bringt nun ein Referenzmuster 34 dort an. Ein entsprechendes Referenzmuster wird auf dem Oberkiefer-Wachswall 22 an der gleichen Stelle angebracht.

Für die Zahnaufstellung werden nun die beiden Referenzmuster virtuell in Übereinstimmung gebracht. Die Höhen- und Seitenlage der Zähne wird damit relativ zu dem Lächelbild festgelegt, und aus der Darstellung gemäß Fig. 6 ist die Einblendung dieses virtuellen Wachswalles in das Lächelbild ersichtlich.

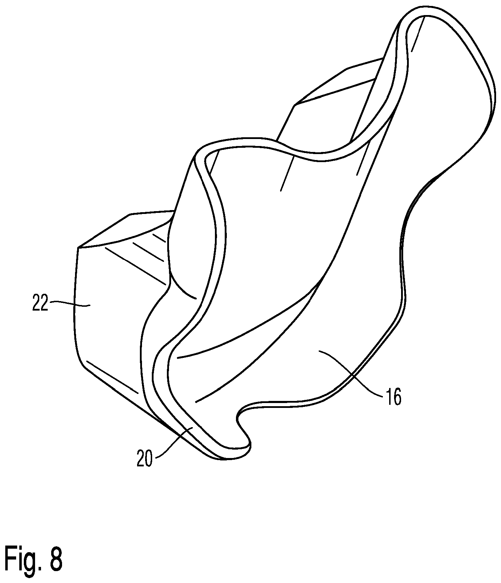

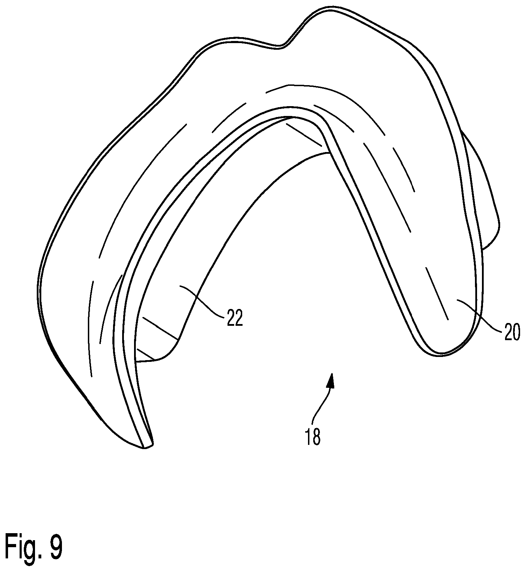

Aus Fig. 7 ist die Anbringung des Referenzmusters 34 auf dem digital gescannten und erstellten Oberkiefer-Wachswall des Patienten ersichtlich. Die Zusammenführung erfolgt zwischen dem 2D-Lächelbild und den 3D-Scandaten der Abformung einschließlich des oder der gescannten Referenzkörper. Das kalibrierte Lächelbild des Patienten wird genutzt, um die 3D-Zahnaufstellung anhand des eingeblendeten Lächelbildes zu kontrollieren und eine sogenannte virtuelle Einprobe der neuen Prothese vorzunehmen. Aus Fig. 8 und 9 sind weitere Ausführungsformen von Abformlöffeln 16 und 18 ersichtlch. Die Abformlöffel bilden je einen im wesentlichen U-Kanal für die Aufnahme des Abformmaterials, so dass, rund ein Stück an die betreffenden Kieferkammbögen 20 sind die Wachswälle 22 angeformt.

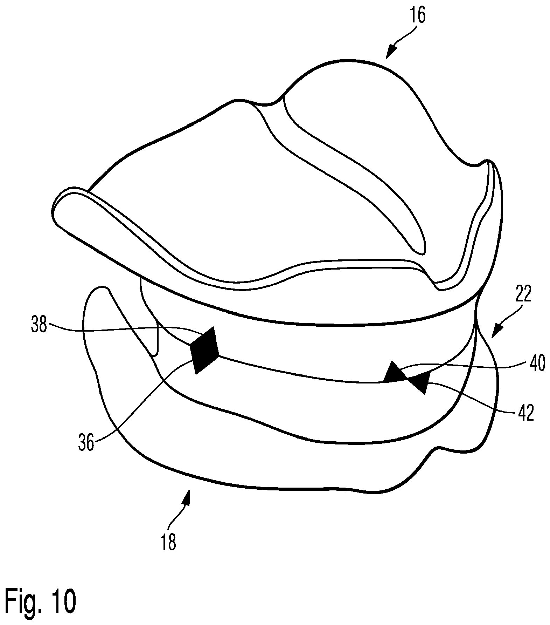

Aus Fig. 10 ist eine weitere Ausführungsform von Abformlöffeln 16 und 18 ersichtlich. Diese weisen an ihren einander zugewandten Flächen, gleichsam den Okklusionsflächen, Aussparungen 36, 38, 40, 42 auf. Diese sind in Fig. 10 zur Sichtbarmachung vestibulär dargestellt, liegen jedoch tatsächlich je in der Okklusionsfläche.

Die Aussparungen sind in an sich bekannten Weise dafür bestimmt, Verschlüsselungsmaterial oder Verschlüsselungskörper aufzunehmen, um so die Abformlöffel 16 und 18 gegeneinander zu verschlüsseln.

## Patentansprüche

1. Verfahren zur Herstellung einer Prothese, insbesondere einer Vollprothese, welche Prothese über eine Abformanordnung hergestellt wird, die Kieferkammbögen mit einander zugewandten Wachswällen aufweist, die gegeneinander insbesondere verschlüsselbar sind, wobei präfabrizierte Abformlöffel (10), die die Kieferkammbögen mit Wachswällen aufweisen, gegebenenfalls durch Erwärmen, manuell hinsichtlich ihrer Größe an die Erfordernisse angepasst werden und
- dass intraoral die Wachswälle aneinander angelegt und das Anlegen überprüft wird und gegebenenfalls die Okklusionsflächen der Wachswälle aneinander angepasst werden,
- dass hieran anschließend die Kieferkammbögen mit Abformmaterial (32) in Abformnuten in an sich bekannter Weise ausgestattet werden und insbesondere eine Funktionsabformung vorgenommen wird,
- dass bei mindestens teilweise eingesetztem Abformlöffel (10) Referenzmuster aufgebracht werden bzw. sind, insbesondere auf dem Oberkieferwachswall oder dem Unterkieferwachswall,
- dass anschließend hieran ein Foto des Patienten mit sichtbarem Referenzmuster gefertigt wird,
- dass gegebenenfalls eine Verschlüsselung über eine Bissregistrierung in an sich bekannter Weise vorgenommen wird,
- dass basierend auf dem insofern benutzten, aus dem Mund des Patienten entnommenen, Abformlöffel (10) ein digitales Modell durch Einscannen der - insbesondere verschlüsselten - Abformung einschließlich des gescannten Referenzmusters angefertigt und aufbereitet wird,
- dass basierend auf den insofern ermittelten Daten die Zahnaufstellung erfolgt,
- dass eine digitale Einprobe der Zahnaufstellung basierend auf dem Foto des Patienten mit dem Referenzmuster (34) vorgenommen wird, und
- dass basierend hierauf die Prothese hergestellt wird, insbesondere durch ein abtragendes oder ein auftragendes Verfahren.

2. Verfahren nach Anspruch 1, **dadurch gekennzeichnet, dass** die Wachswälle des Oberkiefer-Abformlöffel (16) und des Unterkiefer-Abformlöffel (18) hinsichtlich ihrer aufeinander zu weisenden Flächen aneinander angepasst werden, insbesondere unter Bereitstellung von planen Flächen und unter Vermeidung von klaffenden Spalten (30).

3. Verfahren nach einem der vorherigen Ansprüche, **dadurch gekennzeichnet, dass** der Kieferkammbogen (20) bei Körpertemperatur bei Einwirkung einer Kraft im Bereich pysiologisch wirkender Kaukräfte in oraler bzw. bukkaler Richtung plastisch nicht verformt wird, also bei Einwirkung dieser Kraft sich nicht bleibend verformt.

4. Verfahren nach einem der vorherigen Ansprüche, **dadurch gekennzeichnet, dass** ein Referenzmuster (34) auf dem Wachswall (22) und/oder dem Kieferkammbogen (20) angebracht wird und/oder ein Stützstiftregistrat, das in mindestens einen Wachswall (22) eingedrückt wird, die Kieferkammbögen (20) gegeneinander verschlüsselt.

5. Verfahren nach einem der vorherigen Ansprüche, **dadurch gekennzeichnet, dass** an jedem Abformlöffel (10) die Kieferkammnut ausgebildet ist, welche Kieferkammnut von einer oralen Nutseitenflanke und einer bukkalen Nutseitenflanke begrenzt wird, und dass die Nutseitenflanken, insbesondere nach Erwärmung über die Mund-/Körpertemperatur hinaus, durch Einwirkung einer Kraft von weniger als 100 Newton in oraler oder bukkaler Richtung plastisch verformt wird.

6. Verfahren nach einem der vorherigen Ansprüche, **dadurch gekennzeichnet, dass** das in der Kieferkammnut des Abformlöffels (10) aufgenommene Abformmaterial (32) bei Mund-/Körpertemperatur plastisch verformbar wird, und dass das Abformmaterial (32) bei Mund-/Körpertemperatur nachgiebiger als das Material des Kieferkammbogens (20) und/oder des Wachswalls (22) wird.

7. Verfahren nach einem der vorherigen Ansprüche, mit einer Scanvorrichtung und einer Verarbeitungsvorrichtung für die von der Scanvorrichtung bereitgestellten Daten, zur Erzeugung eines gescannten Bildes, mit einem Abformlöffel (10) und einem basierend auf dem Abformergebnis erstellten Modell, wobei mit der Scanvorrichtung das Referenzmuster (34) mit den verformbaren Wachswällen und/oder Kieferkammbögen, insbesondere mit gegeneinander verschlüsselten Oberkiefer- und Unterkiefer-Kammbögen, gescannt wird und wobei mit der Verarbeitungsvorrichtung das Modell hinsichtlich seiner Höhenlage, Seitenlage und/oder Ausrichtung basierend auf dem gescannten Bild hergestellt wird.

8. Verfahren nach einem der vorherigen Ansprüche, **dadurch gekennzeichnet, dass** das Bild, welches das Referenzmuster (34) umfasst, für das Kalibrieren basierend auf dem Referenzmuster (34) eingesetzt wird, und/oder dass das Referenzmuster (34) Ortsidentifikationsmittel aufweist, die die räumliche Lage, insbesondere auch die Winkelstellung, des Referenzmusters (34), bezogen auf die Umgebung, insbesondere die Lippen des Patienten, identifiziert.

9. Verfahren nach einem der vorherigen Ansprüche, **dadurch gekennzeichnet, dass** die Abformanordnung eine Virtualisierungsvorrichtung aufweist, mit welcher basierend auf einem Scanergebnis der insbesondere verschlüsselten Abformung, einschließlich des Referenzmusters (34), die Herstellung eines virtuellen Modells ermöglicht wird, so dass insbesondere ein Bild des Patienten bei leicht geöffneten Lippen einblendbar wird.

10. Verfahren nach einem der vorherigen Ansprüche, **dadurch gekennzeichnet, dass** mit der Verarbeitungsvorrichtung die virtuelle Okklusionsebene basierend auf mindestens einem Bild des Mundes des Patienten festgelegt wird.

11. Verfahren nach einem der vorherigen Ansprüche, **dadurch gekennzeichnet, dass** das Bild des Referenzmusters (34) einem zweidimensionalen Bild des Mundes des Patienten mit geöffneten Lippen im Bereich der Mundöffnung virtuell überlagert wird, und dass die Prothese digital aufstellbar ist, wobei die Aufstellung basierend auf dem Bild kontrollierbar ist.

12. Verfahren nach einem der vorherigen Ansprüche, **dadurch gekennzeichnet, dass** der Abformlöffel mit einem Referenzmuster kombiniert wird, das im Frontbereich auf diesem aufgebracht wird.

13. Verfahren nach einem der vorherigen Ansprüche, **dadurch gekennzeichnet, dass** der Abformlöffel nach Erwärmung, insbesondere in einem Wasserbad auf 50 bis 60 Grad, in bukkal-oraler Richtung, in Anpassung an die Patientenanatomie ggf. auch ockusla-gingival ,für jede Seite des Kieferkammbogens unabhängig von der anderen verformt wird.

14. Verfahren nach einem der vorherigen Ansprüche, **dadurch gekennzeichnet, dass** ein Okklusionom, also ein Gesichtsbogen mit angeformter Bissgabel, so positioniert wird, dass die Bissgabel zwischen beiden Wachswällen zu liegen kommt und dass dadurch die Okklusionsebene definiert wird, und die so erfassten Werte notiert werden.

15. Verfahren nach einem der vorherigen Ansprüche, **dadurch gekennzeichnet, dass** bei geöffneten Mund des Patienten Ästhetiklinien, also mindestens Lippenschlusslinien und Mittellinien, ggf. auch Lachlinien, im labialen Bereich eines Wachswalls, insbesondere des Oberkiefers-Wachswalls angezeichnet werden, und dort Referenzmuster aufgebracht werden, die bei geöffneten Lippen, also beim Lächeln des Patienten, vollständig sichtbar sind.

## Claims

1. A method for producing a prosthesis, especially a full prosthesis, which prosthesis is produced using an impression assembly which has dental ridge arches comprising wax walls facing each other, which wax walls especially are encryptable to one another, wherein prefabricated impression trays (10) comprising the dental ridge arches including wax walls are manually adapted by heating, with respect to their size, to the requirements, if necessary, and
- in that the wax walls are intraorally arranged abutting each another and the arrangement is veryfied and the occlusal surfaces of the wax walls are fitted to each another, if required,
- in that the dental ridge arches are subsequently provided with impression material (32) in impression grooves in a manner known per se and especially a functional impression is taken,
- in that, with the impression tray (10) being at least partially inserted, reference patterns are being applied or are applied, especially onto the upper alveolar wax wall or the lower alveolar wax wall,
- in that a photograph of the patient is subsequently being taken with a reference pattern being visible,
- in that, if necessary, engagement is performed using a bite registration in a manner known per se,
- in that, based on the impression tray (10) used in this respect and removed from the patient's mouth, a digital model is produced and prepared by scanning the - especially engaged - impression including the scanned reference pattern,
- in that the tooth set-up is performed basedon the data determined in this respect,
- in that digital fitting in of the tooth set-up is performed based on the photograph of the patient using the reference pattern (34), and
- in that the prosthesis is produced based thereon, especially by an ablating or an accumulating process.

2. The method according to claim 1, **characterized in that** the wax walls of the upper alveolar impression tray (16) and the lower alveolar impression tray (18) are adapted to each other with regard to their surfaces to be directed towards each other, especially providing flat surfaces and avoiding gaping crevices (30).

3. The method according to one of the preceding claims, **characterized in that** the dental ridge arch (20) will not plastically deform at body temperature under the action of a force in the range of pysiologically acting masticatory forces in the oral or buccal direction, i.e. it does not permanently deform under the action of this force.

4. The method according to one of the preceding claims, **characterized in that** a reference pattern (34) is applied to the wax wall (22) and/or the dental ridge (20), and/or a support pin registration, which is pressed into at least one wax wall (22), encrypts the dental ridge (20) against each other.

5. The method according to one of the preceding claims, **characterized in that** the dental ridge groove is formed on each impression tray (10), which dental ridge groove is delimited by an oral groove side flank and a buccal groove side flank, and that the groove side flanks are plastically deformed, especially after heating beyond the oral/body temperature, by the action of a force of less than 100 Newtons in the oral or buccal direction.

6. The method according to one of the preceding claims, **characterized in that** the impression material (32) accommodated in the dental ridge groove of the impression tray (10) becomes plastically deformable at oral/body temperature, and that the impression material (32) becomes more resilient at oral/body temperature than the material of the dental ridge arch (20) and/or the wax wall (22).

7. The method according to one of the preceding claims, comprising a scanning device and a processing device for the data provided by the scanning device, for generating a scanned image, comprising an impression tray (10) and a model created based on the impression result, wherein the scanning device is used to scan the reference pattern (34) with the deformable wax walls and/or dental ridge arches, especially with upper alveolar and lower dental ridge arches being engaged against each other, and wherein the processing device is used to produce the model with respect to the height position, lateral position and/or orientation thereof based on the scanned image.

8. The method according to one of the preceding claims, **characterized in that** the image comprising the reference pattern (34) is used for calibration based on the reference pattern (34), and/or that the reference pattern (34) comprises location identification means identifing the spatial position, especially also the angular position, of the reference pattern (34) in relation to the environment, especially the lips of the patient.

9. The method according to one of the preceding claims, **characterized in that** the impression assembly comprises a virtualization device, which is used to allow production of a virtual model, such that especially an image of the patient with lips being slightly opened may be displayed, based on a scan result of the especially engaged impression, including the reference pattern (34).

10. The method according to one of the preceding claims, **characterized in that** the virtual occlusal plane is determined using the processing device based on at least one image of the patient's mouth.

11. The method according to one of the preceding claims, **characterized in that** the image of the reference pattern (34) is virtually superimposed on a two-dimensional image of the patient's mouth with lips being open in the region of the mouth opening, and **in that** the prosthesis can be created digitally, wherein creating being controllable based on the image.

12. The method according to one of the preceding claims, **characterized in that** the impression tray is combined with a reference pattern which is applied thereto in the front region.

13. The method according to one of the preceding claims, **characterized in that** the impression tray, after heating, especially in a water bath to 50 to 60 degrees, is deformed in the buccal-oral direction, to adapt to the patient anatomy, and evwntually is also deformed in trhe occlusal-gingival direction, for each side of the alveolar arch independently of each other.

14. The method according to one of the preceding claims, **characterized in that** an occlusionome, i.e. a facial arch with a bite fork being formed thereto, is arranged such that the bite fork will be located between the two wax walls, thereby defining the occlusal plane and recording the values thus accquired.

15. The method according to one of the preceding claims, **characterized in that**, when the patient's mouth is open, esthetic lines, i.e. at least lip closure lines and center lines, eventually also smile lines, are drawn into the labial region of a wax wall, especially the maxillary wax wall, and reference patterns are applied thereto which are completely visible when the patient's lips are open, i.e. when the patient smiles.

## Revendications

1. Procédé de fabrication d'une prothèse, en particulier d'une prothèse complète, où ladite prothèse est produite au moyen d'un arrangement d'empreinte qui présente des crêtes de mâchoire avec des bourrelets de cire se faisant mutuellement face qui peuvent, en particulier, être verrouillées les unes aux autres, où des porte empreintes préfabriqués (10), qui présentent des arcs alvéolaires avec des bourrelets en cire, sont adaptés manuellement par rapport à leur taille aux besoins, si nécessaire par chauffage,
- et en ce qu'à l'intérieur de la bouche les bourrelets de cire sont placées l'une contre l'autre et que le placement est vérifié et, le cas échéant, les surfaces occlusales des bourrelets de cire sont adaptées l'une à l'autre,
- en ce que les arcs alvéolaires sont ensuite équipés de matériau d'empreinte (32) dans des rainures d'empreinte d'une manière connue en soi et, en particulier, une empreinte fonctionnelle est effectuée,
- en ce que, le porte-empreinte (10) étant au moins partiellement inséré, des modèles de référence sont ou seront appliqués, en particulier sur le bourrelet de cire de la mâchoire supérieure ou le bourrelet de cire de la mâchoire inférieure,
∘ en ce qu'une photographie du patient avec un modèle de référence visible est ensuite produite,
∘ en ce que, le cas échéant, un verrouillage est effectué au moyen d'un enregistrement de la morsure d'une manière connue en soi
∘ -en ce que, sur la base du porte-empreinte (10) utilisé à cet effet et prélevé dans la bouche du patient, un modèle numérique est produit et préparé en scannant l'empreinte - en particulier verrouillée - y compris le modèle de référence scanné,
∘ en ce que le montage de la dent est effectuée sur la base des données déterminées dans ce contexte,
∘ en ce qu'un essai numérique de l'installation dentaire est effectué sur la base de la photographie du patient avec le modèle de référence (34), et
∘ en ce que la prothèse est produite sur cette base, notamment par un procédé d'enlèvement ou d'application de matériau.

2. Procédé selon la revendication 1, **caractérisé en ce que** les bourrelets de cire du porte-empreinte de la mâchoire supérieure (16) et du porte-empreinte de la mâchoire inférieure (18) sont adaptées l'une à l'autre par rapport à leurs surfaces opposées, en particulier en fournissant des surfaces planes et en évitant des crevasses (30).

3. Procédé selon l'une des revendications précédentes, **caractérisée en ce que** l'arc de la crête alvéolaire (20) n'est pas plastiquement déformée à la température du corps sous l'action d'une force dans la gamme des forces masticatoires physiologiquement actives dans la direction orale ou buccale, c'est-à-dire qu'elle ne se déforme pas de façon permanente sous l'action de cette force.

4. Procédé selon l'une des revendications précédentes, **caractérisée en ce qu'**un modèle de référence (34) est appliqué sur le bourrelet de cire (22) et/ou sur l'arc de la crête alvéolaire (20) et/ou un modèle de support (34) est appliqué sur le bourrelet de cire (22) et/ou un enregistrement de la broche de support, qui est enfoncé dans au moins une crête de cire (22), verrouille les arcs de la crête alvéolaire (20) les uns contre les autres.

5. Procédé selon l'une des revendications précédentes, **caractérisée en ce que** le sillon de crête alvéolaire est formé sur chaque porte-empreinte (10), lequel sillon de crête alvéolaire étant délimité par un flanc latéral de sillon oral et un flanc latéral de sillon buccal, et **en ce que** les flancs latéraux de sillon sont déformés plastiquement, en particulier après chauffage au-delà de la température orale/corporelle, par l'action d'une force de moins de 100 Newtons dans la direction orale ou buccale.

6. Procédé selon l'une des revendications précédentes, **caractérisé en ce que** le matériau d'empreinte (32) logé dans le sillon de la crête alvéolaire du porte-empreinte (10) devient plastiquement déformable à la température de la bouche/corps, et que le matériau d'empreinte (32) devient plus souple à la température de la bouche/corps que le matériau de l'arc de la crête alvéolaire (20) et/ou du bourrelet de cire (22).

7. Procédé selon l'une des revendications précédentes, comprenant un dispositif de balayage et un dispositif de traitement des données fournies par le dispositif de balayage, pour générer une image numérisée, comprenant un porte-empreinte (10) et un modèle créé sur la base du résultat de l'empreinte, où le dispositif de balayage est utilisé pour balayer le modèle de référence (34) avec les bourrelets de cire déformables et/ou les crêtes de mâchoire, en particulier avec les crêtes de mâchoire supérieure et de mâchoire inférieure verrouillées l'une contre l'autre, et où le dispositif de traitement est utilisé pour produire le modèle en ce qui concerne sa position en hauteur, sa position latérale et/ou son orientation sur la base de l'image balayée.

8. Procédé selon l'une des revendications précédentes, **caractérisée en ce que** l'image comprenant le modèle de référence (34) est utilisée pour le calibrage basé sur le modèle de référence (34), et/ou que le modèle de référence (34) comprend des moyens d'identification de remplacement qui identifient la position spatiale, en particulier également la position angulaire, du modèle de référence (34), par rapport à l'environnement, en particulier les lèvres du patient.

9. Procédé selon l'une des revendications précédentes, **caractérisé en ce que** le dispositif d'empreinte comprend un dispositif de virtualisation, avec lequel, sur la base d'une résultat de balayage de l'empreinte en particulier verrouillée, y compris le modèle de référence (34), la production d'un modèle virtuel est rendue possible, de manière à pouvoir afficher en particulier une image du patient avec les lèvres légèrement ouvertes.

10. Procédé selon l'une des revendications précédentes, **caractérisée en ce qu'**avec le dispositif de traitement le plan d'occlusion virtuel est défini à partir d'au moins une image de la bouche du patient.

11. Procédé selon l'une des revendications précédentes, **caractérisée en ce que** l'image du modèle de référence (34) est virtuellement superposée à une image bidimensionnelle de la bouche du patient avec des lèvres ouvertes dans la région de l'ouverture de la bouche, et **en ce que** la prothèse peut être mise en place de manière numérique, où la mise en place peut être contrôlé sur la base de l'image.

12. Procédé selon l'une des revendications précédentes, **caractérisée en ce que** le porte-empreinte est combiné avec un modèle de référence qui lui est appliqué dans la région antérieure.

13. Procédé selon l'une des revendications précédentes, **caractérisé en ce que** le porte-empreinte, après avoir été chauffé, en particulier dans un bain-marie à 50 à 60 degrés, est déformé dans le sens bucco-oral, en adaptation à l'anatomie du patient éventuellement aussi occlusal-gingival, pour chaque côté de l'arcade de crête alvéolaire indépendamment de l'autre.

14. Procédé selon l'une des revendications précédentes, **caractérisée en ce qu'**un occlusionome, c'est-à-dire un arc facial sur lequel est formée une fourchette buccale, est positionné de telle manière que la fourchette à mordre vienne se placer entre les deux bourrelets de cire et que le plan d'occlusion soit ainsi défini, et les valeurs ainsi enregistrées sont notées.

15. Procédé selon l'une des revendications précédentes, **caractérisée en ce que**, lorsque la bouche du patient est ouverte, des lignes esthétiques, c'est-à-dire au moins des lignes de fermeture des lèvres et des lignes centrales, éventuellement aussi des lignes de sourire, sont tracées dans la région labiale d'une bourrelet de cire, en particulier du bourrelet de cire maxillaire, et des modèles de référence y sont appliqués qui sont complètement visibles lorsque les lèvres du patient sont ouvertes, c'est-à-dire lorsque le patient sourit.
